Europäisches Patentamt

European Patent Office

Office européen des brevets

(11) Veröffentlichungsnummer: **0 277 556**
**A2**

## (12) EUROPÄISCHE PATENTANMELDUNG

(21) Anmeldenummer: 88100896.5

(22) Anmeldetag: 22.01.88

(51) Int. Cl.⁴: **C07D 265/30** , C07C 129/12 , C07C 129/16 , C07D 233/64 , C07D 233/58 , C07D 239/04 , C07C 87/30 , C07D 213/20 , C07C 87/123 , A01N 43/00 , A01N 33/02

(30) Priorität: 31.01.87 DE 3702932

(43) Veröffentlichungstag der Anmeldung:
10.08.88 Patentblatt 88/32

(84) Benannte Vertragsstaaten:
AT BE CH DE ES FR GB GR IT LI NL SE

(71) Anmelder: Dr. Wolman GmbH
Dr.-Wolman-Strasse 31-33
D-7573 Sinzheim(DE)

(72) Erfinder: Goettsche, Reimer, Dr.
Waldstrasse 27
D-7570 Baden-Baden 19(DE)
Erfinder: Marx, Hans-Norbert
Mozartweg 8
D-7580 Buehl-Weitenung(DE)

(74) Vertreter: Schweiss, Werner, Dr. et al
BASF Aktiengesellschaft Patentabteilung
Carl-Bosch-Strasse 38
D-6700 Ludwigshafen(DE)

(54) Salze von stickstoffhaltigen organischen Verbindungen mit Fluoroborsäure und diese enthaltende Holzschutzmittel.

(57) Wasserunlösliches Salz einer organischen stickstoffhaltigen Verbindung mit Fluorborsäure außer dem Salz des N-Tridecyl-2,6-dimethylmorpholins oder des 2-(Methoxy-carbonylamino)-benzimidazols und das wasserunlösliche Salz enthaltende Fungizide.

EP 0 277 556 A2

## Salze von stickstoffhaltigen organischen Verbindungen mit Fluoroborsäure und diese enthaltende Holzschutzmittel

Die vorliegende Erfindung betrifft neue Salze der Fluoroborsäure mit Stickstoffverbindungen und diese enthaltende Holzschutzmittel.

Es ist bekannt, das Salz des 2-(Methoxy-carbonylamino)-benzimidazols mit Fluoroborsäure als Fungizid im Holzschutz zu verwenden (DE-3 138 575.3). Durch die Fluoroborsäure wird ein zusätzlicher fungizider Wirkstoff in das Benzimidazolderivat eingeführt und das wasserunlösliche Benzimidazolderivat wird in das wasserlösliche Salz überführt.

Ein älterer Vorschlag betrifft das Fluoroborat des N-Tridecyl-2,6-dimethylmorpholins und seine Anwendung als Fungizid und als Holzschutzmittel (DE-3 605 007.5).

Es ist ferner bekannt, wasserlösliche Salze der Fluoroborsäure mit Alkalidichromat im Holz zur Umsetzung zu bringen und dadurch das Holz zu - schützen. Diese Salze sind als CFB-Salze im Handel.

Die wasserlöslichen Salze der Fluoroborsäure werden im Holz nicht festgelegt (nicht fixiert).

Sie können nur durch Zusatz von Chromaten im Holz über Chrom-Kryolithbildung (z.B. $Na_3CrF_6$) fixiert werden. Diese Fluorfixierung ist jedoch nicht vollkommen, da sich die freien Fluoridionen aus den Fluoroboraten erst bei pH-Werten über pH 7 im Holz bilden und hierzu ein erheblicher Chromatüberschuß notwendig ist. Bei den üblichen Salzgemischen (ca. 33 % Alkalidichromat) wird über 50 % des Fluors mit Wasser ausgewaschen. Die Salzgemische benötigen für die Fixierung außerdem 2 bis 6 Wochen je nach Klimabedingungen, hierbei kann Chrom (VI) aus dem Holz ausgewaschen werden und dadurch die Umwelt belasten.

Darüber hinaus ergeben sich bei der Verbrennung der so imprägnierten Hölzer durch den Chromgehalt Probleme, denn bei der Verbrennung wird Chrom III zur Chrom VI oxidiert, das Chrom VI kann dann durch Regen aus der Asche ausgewaschen werden.

Es wurde nun gefunden, daß wasserunlösliche Salze von organischen stickstoffhaltigen Verbindungen mit Fluoroborsäure außer dem Salz des N-Tridecyl-2,6-dimethylmorpholins oder des 2-(Methoxy-carbonylamino)-benzimidazols eine gute fungizide und insektizide Wirkung haben und bei der Verwendung als Holzschutzmittel nur wenig aus dem Holz ausgewaschen werden.

Wasserunlösliche Salze sind Salze, die zu weniger als 1 % (Gewichtsprozent) im Wasser löslich sind, d.h. in 1 Liter Wasser lösen sich weniger als 10 g der Salze. Insbesondere sind es Salze, die sich zu weniger als 1 Promille im Wasser lösen, d.h. in 1 Liter Wasser lösen sich weniger als 1 g der Salze.

Organische stickstoffhaltige Verbindungen sind z.B. Verbindungen mit einem oder mehreren Stockstoffatomen, primäre, sekundäre oder tertiäre Amine, Polyamine oder heterocyclische Verbindungen oder quartäre Ammonium-Verbindungen.

Es werden beispielsweise folgende Verbindungen genannt:

Di-2-ethyl-hexylamin, Di-decylamin, N-Methyl,N,N-didecylamin, N,N-Di-methyl-N-$C_{12}$/$C_{14}$-alkylamin, Morpholinderivate, 4-(3-(p-tertiär-Butyl-phenyl)-2-methyl-propyl)-2,6-cis-dimethylmorpholin, 4-(n-Dodecyl)-2,6-di-methylmorpholin, 4-Cyclododecyl-2,6-dimethylmorpholin, Imidazol-, Guanidin-, Pyridin-, Chinaldin-, Pyrrolidin-, Piperazin-, Piperidinderivate. Quartäre Ammoniumverbindungen, die der allgemeinen Formel $(R^1R^2R^3R^4N)$ Z entsprechen, wobei

$R^1$ einen Alkylrest mit 8 bis 20 Kohlenstoffatomen, insbesondere einen Alkylrest mit 12 bis 20 Kohlenstoffatomen, oder einen Benzylrest bedeutet, der gegebenenfalls durch $C_1$-$C_{20}$-Alkyl oder Halogen substituiert ist.

$R^2$ $C_1$-$C_6$-Alkyl, $C_1$-$C_4$-Alkoxy oder $C_3$-$C_9$-Alkoxyalkyl, $R^3$ $C_1$-$C_6$-Alkyl oder $C_1$-$C_4$-Alkoxy und $R^4$ $C_1$-$C_{20}$-Alkyl oder $C_1$-$C_4$-Alkoxy bedeuten, oder je zwei der Reste $R^1$ und $R^4$ zusammen mit dem Stickstoffatom einen heterocyclischen Rest bilden, der 4 bis 5 C-Atome, 1 bis 2 N-Atome und eine, zwei oder drei Doppelbindungen enthält, wobei die Kohlenstoffatome gegenbenenfalls durch $C_1$-$C_4$-Alkyl oder Halogen substituiert sind, und Z einen Säurerest z.B. Halogenid (Chlorid) bedeutet.

Die gute insektizide Wirkung der Salze zeigt sich beispielsweise gegenüber Hausbockeilarven. Die gute fungizide Wirkung zeigt sich gegenüber Holz schädigenden Pilzen.

Die in Wasser unlöslichen Fluoroborate können mit Hilfe von ionischen oder nicht ionischen Emulgatoren, gegebenenfalls unter Zusatz von organischen Lösungsmitteln, zu Emulsionskonzentraten formuliert werden, die in Wasser klare Mikroemulsionen bilden. Diese Mikroemulsionen können dann durch übliche handwerkliche Verfahren (z.B. Streichen, Tauchen, Sprühen) oder großtechnische Verfahren (Kesseldruckverfahren) ins Holz gebracht werden und bewirken einen ausreichenden Schutz, insbesondere gegen tierische Holzzerstörer, auch im Außenbereich, da sie überraschend durch Witterungseinflüsse (Regen) nicht oder nur gering ausgewaschen werden. Hierdurch kann auf die Verwendung von Chrom VI-Verbindungen als umweltgefährdende Komponente verzichtet werden.

Die Herstellung der Salze kann z.B. in der Weise erfolgen, daß die organischen, stickstoffhaltigen Basen oder ihre Salze mit äquivalenten Mengen von Fluoroborsäure oder wasserlöslichen Fluoroboraten (z.B. Natriumfluoroborat) in Wasser zur Reaktion gebracht werden, das sich bildende Fluoroborat fällt aus und wird abfiltriert und kann gegebenenfalls anschließend getrocknet werden. Die Fluoroborate können dann mit organischen Lösungsmitteln und nicht-ionischen Emulgatoren gemischt und die Mischung in Wasser emulgiert werden.

Eine einfachere Methode wasseremulgierbare Mischungen herzustellen besteht z.B. darin, daß stickstoffhaltige Verbindungen oder ihre Salze, z.B. nach Zusatz von Dimethyl-$C_{10}$-$C_{18}$-alkylaminen und/oder N-$C_{10}$-$C_{18}$-Alkyl-1,3-di-aminopropan, mit Fluoroborsäure, gegebenenfalls unter Zusatz von substituierten Carbonsäuren, z.B. Milchsäure, Weinsäure, Glykolsäure, Methoxypropionsäure, Methoxyessigsäure, Adipinsäure und/oder anorganischen Säuren z.B. Phosphorsäure, Hypophosphoshorsäure, Phosphonsäuren umgesetzt oder neutralisiert werden, so daß die Salze des Dimethylalkylamins oder N-Alkyl-1,3-diaminopropans emulgierend auf die Fluoroborate wirken. Mit Wasser entstehen aus diesen Mischungen wäßrige Mikroemulsionen. Durch die eigene fungizide Wirksamkeit des Dimethylalkylamins oder des N-Alkyl-1,3-diaminopropans wird die fungizide Wirksamkeit der Salze zusätzlich verbessert. Die Zugabe an Fluoroborsäure kann in äquivalenter Menge, im Überschuß (z.B. bis zu 50 %) aber auch im Unterschuß (z.B. bis zu 30 %) erfolgen, wobei die fehlende Säure z.B. durch die oben genannten Säuren ergänzt wird.

Die Dimethylalkylaminosalze bzw. N-Alkyl-1,3-diaminopropansalze können auch in Mischung mit organischen Lösungsmitteln oder nicht ionischen oder anderen ionischen Emulgatoren den Fluoroboraten zugesetzt werden. Es ist überraschend, daß die Fluoroborate in einfacher Weise in Wasser emulgiert werden können, wobei klare Mikroemulsionen entstehen.

Zur Verbesserung des Korrosionsverhaltens gegenüber Eisen und Stahl können gegebenenfalls Carbonsäuren, z.B. Isononansäure, Isooctansäure, oder andere Korrosionsinhibitoren zugesetzt werden. `

Statt Fluoroborsäure oder ihrer wasserlöslichen Salze kann für die Herstellung der Salze auch eine Mischung von wasserlöslichen Fluoriden mit Borsäure oder Boraten verwendet werden.

Die fungizide und/oder insektizide Wirksamkeit kann durch Zusatz von bekannten wasserunlöslichen, organischen Fungiziden und/oder Insektiziden, z.B. N-Cyclohexyl-N-methoxy-2,5-dimethylfurancarbonsäureamid, Jodbenzoesäureanilid, Permethrin oder Lindan erweitert und verbessert werden. Die emulgierende Wirkung der eingesetzten Emulgatoren reicht meist aus, um die obengenannten Fungizide und Insektizide bei Zugabe von Wasser in Mikroemulsionen zu überführen, gegebenenfalls ist die Zugabemenge an Emulgatoren zu erhöhen.

Den fluoroborhaltigen Mikroemulsionen können zur Verbesserung der Wirksamkeit gegen Bläue auch wasserlösliche Salze des 2-(Methoxy-carbonylamino)-benzimidazols zugesetzt werden.

Geeignete Mischungen für Mikroemulsionen enthalten beispielsweise (Gew.%)

15 bis 30 %, vorzugsweise 18 bis 27 % einer organischen stickstoffhaligen Verbindung,

5 bis 25 %, vorzugsweise 8 bis 15 % Fluoroborsäure,

15 bis 45 %, vorzugsweise 20 bis 35 % Dimethylalkylamin oder Alkyldiaminopropan,

7 bis 35 %, vorzugsweise 15 bis 30 % substituierte Carbonsäure oder anorganische Säure,

0 bis 15 % weiterer wasserunlöslicher organischer Wirkstoff,

0 bis 20 % organisches Lösungsmittel,

2,5 bis 20 %, vorzugsweise 5 bis 13 % Wasser,

wobei die Summe jeweils 100 % (Gew.%) ergibt.

Die Erfindung erstreckt sich gleichermaßen auf die Mischungen (Konzentrate) wie auf die durch Emulgieren in Wasser hergestellten Mikroemulsionen entsprechend geringer Einzelkonzentration. Die folgenden Beispiele erläutern Mischungen, die in Wasser zu Mikroemulsionen emulgiert werden können.

Beispiel 1 (Vergleich - bekannt)

Holzschutzmittel:

Handelsübliche wasserlösliche CFB-Salze (Mischung wasserlöslicher Salze auf Basis Chrom, Fluor, Bor)

Gehalt: 11,5 % Cr (Gewichtsprozent) .
2,6 % B
16,5 % F

Prüfklötzchen (Abmessung 5,0 x 2,5 x 1,5 $cm^3$) aus Kiefernsplintholz wurden 3 Minuten in eine 10prozentige CFB-Salzlösung getaucht, anschließend 4 Wochen in feuchter Atmosphäre langsam abgetrocknet. Die Auswaschung erfolgte durch mehrmaliges Waschen mit Wasser. Chrom und Fluor wurden im Auswaschwasser analytisch bestimmt (jeweils 10 Prüfklötzchen wurden zusammen ausgewaschen).

Chromauswaschung: 27,2 %
Fluorauswaschung: 70 %

Beispiel 2 (Vergleich - bekannt)

Holzschutzmittel: NaBF₄ (wasserlöslich)
Anwendungskonzentration: 10 %
Tauchzeit: 3 Minuten
10 Parallel-Versuche
Auswaschung nach 4 Wochen Lagerung
Fluorauswaschung: 96 %

Beispiel 3 Morpholinderivate

3.1 Fenpropemorphfluoroborat = 4-(3-(p-tert.-Butylphenyl)-2-methylpropyl)-2,6-cis-dimethylmorpholinfluoroborat 22 % Fenpropemorph
30 % Cocosdimethylamin (C₁₂/C₁₄) = N,N-Dimethyl-N-C₁₂/C₁₄-alkyl-amin
9 % Fluoroborsäure
29 % Milchsäure
10 % Wasser

(Fluoroborsäure im Überschuß: ca. 40 %)
Anwendungskonzentration in Wasser: 5 %
Tauchzeit: 3 Minuten
In Vorversuchen war eine Wirksamkeit bei dieser Konzentration gegen Hausbocklarven nach Auswaschung festgestellt worden.
Fixierung: 4 Wochen
Auswaschung: 30 % Fluor
(Es wurde praktisch nur der Überschuß an Fluoroborsäure ausgewaschen)

3.2 Aldimorphfluoroborat = 4-n-Dodecyl-2,6-dimethylmorpholinfluoroborat 20 % Aldimorph
30 % Cocosdimethylamin (C₁₂/C₁₄)
10 % Fluoroborsäure
26 % Milchsäure
14 % Wasser

(Fluoroborsäure im Überschuß: ca. 60 %)
Anwendungskonzentration in Wasser: 5 %
Tauchzeit: 3 Minuten
Fixierung: 4 Wochen
Auswaschung: 36 % Fluor

3.3 Dodemorphfluoroborat = 4-Cyclododecyl-2,6-dimethylmorpholinfluoroborat 20 % Dodemorph
30 % N-C₁₂/C₁₄-Alkyl-1,3-diaminopropan
17 % Milchsäure
10 % Fluoroborsäure
13 % Wasser

Beispiel 4 Guanidinderivate

4.1 Dodecylguanidinfluoroborat 20 % Dodecylguanidinacetat (Dodine)
30 % Dodecyldimethylamin
27 % Milchsäure
10 % Fluoroborsäure
8 % Wasser
5 % Butylglykol
(Fluorborsäure im Überschuß)

Anwendungskonzentration in Wasser: 5 %
Tauchzeit: 3 Minuten
Fixierung: 4 Wochen
Auswaschung: 30 % Fluor

4.2 Chlorhexidinfluoroborat 1,6-Di-(4-Chlorphenyldiguanidino)-hexan-fluoroborat 20 % Chlorhexidin
30 % Dimethyldodecylamin
30 % Methoxypropionsäure
10 % Fluoroborsäure
10 % Wasser

Anwendungskonzentration in Wasser: 5 %
Tauchzeit: 3 Minuten
Fixierung: 4 Wochen
Auswaschung: 17 % Fluor

4.3 20 % Bis(8-guanidin-octyl)-amintriacetat (Guazatin)
30 % Cocosdimethylamin (C₁₂/C₁₄)
10 % Fluoroborsäure
27 % Milchsäure
13 % Wasser

Beispiel 5 Imidazolderivate

5.1 Imazalil-fluoroborat

1-(2-(2,4-Dichlorpheny)-2-(2-propenyloxyethyl)-H-imidazolfluoroborat 20 % Imazalil
30 % Cocos-1,3-diaminopropan
30 % Milchsäure
10 % Fluoroborsäure
10 % Wasser

Anwendungskonzentration: 5 %
Tauchzeit: 3 Minuten
Fixierung: 4 Wochen
Auswaschung: 27 % Fluor

5.2 N-Dodecylimidazolfluoroborat 20 % N-Dodecylimdazol
30 % Cocos-1,3-diaminopropan
30 % Milchsäure
10 % Fluoroborsäure
10 % Wasser

Anwendungskonzentration: 5 %
Tauchzeit: 3 Minuten
Fixierung: 4 Wochen
Auswaschung: 20 % Fluor

5.3 Glyodinfluoroborat = 2-Heptadecyl-2-imidazolinfluoroborat 20 % Glyodin
30 % Cocos-1,3-diaminopropan
30 % Milchsäure
10 % Fluoroborsäure
10 % Wasser

Beispiel 6 Pyrimidinderivate

Hexatidinfluoroborat 1,3-Bis(2-ethylhexyl)-5-amino-5-methylhexahydropyrimidinfluoroborat 20 % Hexa-tidin
30 % Alkyldimethylamin $(C_{12}/C_{14}/C_{16})$ = N-$C_{12}$,$C_{14}$,$C_{16}$-Alkyl-N,N-dimethylamine
30 % Milchsäure
10 % Fluoroborsäure
10 % Wasser

Anwendungskonzentration in Wasser: 5 %
Tauchzeit: 3 Minuten
Fixierung: 4 Wochen
Auswaschung 26 % Fluorid

Beispiel 7 Quartäre Ammoniumverbindungen

7.1 N-Dodecylpyridiniumfluoroborat 25 % N-Dode-cylpyridiniumchlorid
30 % Dodecyldimethylamín
10 % Milchsäure
10 % Fluoroborsäure
10 % Isopropanol
15 % Wasser

Anwendungskonzentration: 5 %

Tauchzeit: 3 Minuten
Fixierung: 4 Wochen
Auswaschung: 27 % Fluor

7.2 25 % Benzalkoniumchlorid $(C_{12}/C_{14})$ = N-Benzyl-N-$C_{12}$,$C_{14}$-alkyl-N,N,-dimethylammoniumchlorid
40 % Dodecyldimethylamin
10 % Methoxyessigsäure
10 % Fluoroborsäure
5 % Ethanol
10 % Wasser

5pronzentig in Wasser klare Mikroemulsion.

Beispiel 8

8.1 Di-2-ethylhexylaminfluoroborat 20 % Di-2-ethyl-hexylamin
30 % N-Cocos-1,3-diaminopropan
27 % Milchsäure
10 % Fluoroborsäure
13 % Wasser

Anwendungskonzentration in Wasser: 5 %
Tauchzeit: 3 Minuten
Fixierung: 4 Wochen
Auswaschung: 17 % Fluor

8.2 Di-decyl-aminfluoroborat 20 % Di-decylamin
30 % N-Cocos-1,3-diaminopropan
27 % Milchsäure
10 % Fluoroborsäure
13 % Wasser

8.3 Methyldidecylaminfluoroborat 20 % Methyldidecylamin
30 % N-Cocos-1,3-diaminopropan
27 % Milchsäure
10 % Fluoroborsäure
13 % Wasser

Beispiel 9

Beispiel mit zusätzlichem Wirkstoff 20 % Chlorhe-xidin
30 % Cocosdimethylamin $(C_{12}/C_{14})$
17 % Milchsäure
10 % Fluoroborsäure
10 % N-Cyclohexyl-N-methoxy-2,5-dimethylfuran-3-carbonsäureamid
13 % Wasser

5pronzentige Anwendungskonzentration in Wasser: klare Mikroemulsion

Es können auch andere Carbonsäuren, z.B. Zitronensäure, Weinsäure, Adipinsäure verwendet werden. Für die einfache Herstellung der Mischungen empfiehlt sich jedoch die Anwendung von Säuren, die handelsüblich in flüssiger Form vorliegen (z.B. Milchsäure 90 %). Solche Mischungen in Form von Lösungen sind durch Mischen der Einzelbestandteile sofort erhältlich. Bei kristallinen Säuren (wie zuvor genannt) sind zur Herstellung von Lösungen lange Rührzeiten erforderlich. Die Fluoroborsäure kann auch im Überschuß vorliegen, durch Diffusion werden dann solche Bereiche des Holzes geschützt, die einer Imprägnierung nicht zugänglich sind (Kernholz).

Es empfiehlt sich, je nach Säurezugabe und Anwendungskonzentration einen pH-Wert von 1,5-6,0, vorzugsweise 2,0-4,0 in der Mischung einzustellen.

Die Mischungen können durch wasserlösliche Kontrollfarbstoffe eingefärbt werden. Durch wasserunlösliche Farbstoffe, die sich in den Mischungen lösen und mit diesen emulgiert werden, lassen sich witterungsbeständige Anfärbungen des Holzes erzielen.

Beispiel 10

Holzschutzmittel mit zusätzlicher Bläuewirksamkeit
16,75 % Imazalil
25,0 % Cocosdimethylamin
15,0 Milchsäure
25,0 % Fluoroborsäure
7,5 % Hypophosphorige Säure
4,25 % 2-(Methoxy-carbonylamino)-benzimidazol
Klare Mikroemulsion in Wasser (5 %).

**Ansprüche**

1. Wasserunlösliches Salz einer organischen stickstoffhaltigen Verbindung mit Fluorborsäure außer dem Salz des N-Tridecyl-2,6-dimethylmorpholins oder des 2-(Methoxy-carbonylamino)-benzimidazols.

2. Fungizid, enthaltend einen festen oder flüssigen Trägerstoff und ein wasserunlösliches Salz einer organischen stickstoffhaltigen Verbindung mit Fluorborsäure außer dem Salz des N-Tridecyl-2,6-dimethylmorpholins oder des 2-(Methoxy-carbonylamino)-benzimidazols.

3. Holzschutzmittel, enthaltend einen festen oder flüssigen Trägerstoff und ein wasserunlösliches Salz einer organischen stickstoffhaltigen Verbindung mit Fluorborsäure außer dem Salz des N-Tridecyl-2,6-dimethylmorpholins oder des 2-(Methoxy-carbonylamino)-benzimidazols.

4. Verfahren zur Bekämpfung von Pilzen, dadurch gekennzeichnet, daß man die Pilze oder die durch Pilzbefall bedrohten Materialien mit einem wasserunlöslichen Salz einer organischen stickstoffhaltigen Verbindung mit Fluorborsäure außer dem Salz des N-Tridecyl-2,6-dimethylmorpholins oder des 2-(Methoxy-carbonylamino)-benzimidazols behandelt.

5. Verfahren zur Bekämpfung von Insekten, dadurch gekennzeichnet, daß man die Insekten oder die durch Insektenbefall bedrohten Materialien mit einem wasserunlöslichen Salz einer organischen stickstoffhaltigen Verbindung mit Fluorborsäure außer dem Salz des N-Tridecyl-2,6-dimethylmorpholins oder des 2-(Methoxy-carbonylamino)-benzimidazols behandelt.

6. Mischung, enthaltend ein wasserunlösliches Salz einer organischen stickstoffhaltigen Verbindung mit Fluorborsäure außer dem Salz des N-Tridecyl-2,6-dimethylmorpholins oder des 2-(Methoxy-carbonylamino)-benzimidazols und das Salz einer Dimethyl-$C_{10}$-$C_{16}$-alkylamin-mischung mit einer substituierten Carbonsäure.

7. Mikroemulsion, hergestellt durch Emulgieren einer Mischung gemäß dem vorhergehenden Anspruch mit Wasser.